(19) **Europäisches Patentamt European Patent Office Office européen des brevets**

(11) **EP 3 576 711 B1**

(12) **EUROPEAN PATENT SPECIFICATION**

(45) Date of publication and mention of the grant of the patent:
**13.04.2022 Bulletin 2022/15**

(21) Application number: **18706069.4**

(22) Date of filing: **05.02.2018**

(51) International Patent Classification (IPC):
*A61K 8/06* *(2006.01)*     *A61K 8/81* *(2006.01)*
*A61K 8/58* *(2006.01)*     *A61K 8/02* *(2006.01)*
*A61Q 19/00* *(2006.01)*

(52) Cooperative Patent Classification (CPC):
**A61K 8/064; A61K 8/0241; A61K 8/585; A61K 8/8152; A61Q 19/007;** A61K 2800/412; A61K 2800/546

(86) International application number:
**PCT/US2018/016841**

(87) International publication number:
**WO 2018/144994 (09.08.2018 Gazette 2018/32)**

(54) **PERSONAL CARE COMPOSITION**

**KÖRPERPFLEGEZUSAMMENSETZUNG**

**COMPOSITION DE SOINS PERSONNELS**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2017 US 201715425713**

(43) Date of publication of application:
**11.12.2019 Bulletin 2019/50**

(73) Proprietor: **The Procter & Gamble Company Cincinnati, OH 45202 (US)**

(72) Inventor: **SUNKEL, Jorge, Max Cincinnati Ohio 45202 (US)**

(74) Representative: **P&G Patent Belgium UK N.V. Procter & Gamble Services Company S.A. Temselaan 100 1853 Strombeek-Bever (BE)**

(56) References cited:
**EP-A1- 3 114 962          WO-A1-2017/004108**
**WO-A2-2008/155228       US-A1- 2013 243 836**
**US-A1- 2015 224 046      US-A1- 2015 342 864**

Remarks:
The file contains technical information submitted after the application was filed and not included in this specification

**Description**

FIELD

The present application relates generally to a personal care composition in the form of a water-in-oil, high internal phase emulsion that includes a non-thickening superabsorbent polymer. The shear thinning behavior of the high internal phase emulsion in combination with the superabsorbent polymer provides improved product aesthetics.

BACKGROUND

A variety of products are available to consumers to provide skin care benefits and to counteract signs of skin aging (e.g., fine lines, wrinkles, and uneven skin texture and tone). To be effective, these products should be applied regularly and over a suitable treatment period. To encourage the desired or ideal usage, it is important for the product to have a desirable appearance and a pleasant feel when applied. In addition, any skin care actives in the personal care composition should be adequately delivered to provide the desired skin health or appearance benefit.

It is not uncommon for a personal care composition to be in the form of an emulsion. Emulsions can provide formulation flexibility and a wide range of compositional properties. Emulsions are generally formed by mixing two or more immiscible liquids together such that they form a single composition with two or more discrete phases. In a conventional two-phase emulsion system, a discontinuous phase (sometimes referred to as the internal phase or dispersed phase) is dispersed throughout a continuous phase as a multitude of droplets. In cosmetics, the two most common types of emulsions are oil-in-water (O/W) emulsions and water-in-oil (W/O) emulsions.

O/W emulsions have a continuous aqueous phase that typically includes water, water miscible liquids, and/or water soluble materials. The continuous aqueous phase of an O/W emulsion typically accounts for more than 50% of the volume of the composition. The relatively high water content of a O/W emulsion provides users with a sensory perception of lightness and/or coolness when applied to skin. In addition, O/W emulsions can provide a cost advantage to manufacturers, since water is a relatively inexpensive material. Formula flexibility is another advantage of O/W emulsions. Many of the commonly used ingredients in personal care composition are water soluble, and thus the relatively large amount of water in an O/W emulsion composition allows for the inclusion of a wide range and/or amount of water soluble actives.

While O/W emulsions can provide a desirable light, cool feel when applied to skin, there is a perception by at least some consumers that light-feeling skin care products formed from O/W emulsions, such as lotions and serums, do not moisturize as well as thicker, heavier skin creams. In addition, electrolytic cosmetic active ingredients (e.g., salicylic acid, salicylate, water-soluble sunscreens, vitamin C, and citric acid) can destabilize O/W emulsions. As a result, it may be necessary to include additional ingredients such as thickeners, co-emulsifiers, and/or other ingredients to an O/W emulsion, which can increase formulation cost/complexity and/or undesirably impact the sensory properties of the composition (e.g., cause tackiness).

W/O emulsions are commonly used to avoid the drawbacks of O/W emulsions. The continuous oil phase of a W/O emulsion typically accounts for more than 50% of the volume of the composition. Skin care products in the form of a W/O emulsion are generally perceived as providing a smooth, silky, non-tacky feel when applied to skin and good moisturization after application. W/O emulsions may also have reduced formulation complexity, as compared to O/W emulsions, because fewer ingredients may be needed to stabilize the emulsion. Further, a wide variety of skin moisturizing agents such as emollients, which tend to be oils, can be more readily incorporated into the continuous oil phase of a W/O emulsion.

However, because the oil phase of a W/O emulsion is typically a large portion of the composition, the composition may feel undesirably heavy and/or greasy to some users (e.g., users who prefer the lighter feel of lotions and serums). In addition, the oils used in the continuous phase of a W/O emulsion may be more expensive than water, and thus may present economic disadvantages compared to O/W emulsions. Accordingly, it would be desirable to provide a personal care composition in the form of an emulsion that provides the benefits of O/W and W/O emulsions, but without at least some of the corresponding disadvantages.

In some instances, high internal phase emulsions (HIPEs) can provide the advantages of low (or medium) internal phase O/W and W/O emulsions, while minimizing or even eliminating some of the disadvantages. HIPEs include a dispersed phase present at an amount greater than the continuous phase. For example, the volume fraction of the dispersed phase of a HIPE may be greater than 0.74. In some instances, skin care compositions in the form of a W/O HIPE can provide the desirable light feel of a O/W emulsion and the perception of good moisturizing associated with a W/O emulsion.

However, HIPEs also have some disadvantages. For example, when a W/O HIPE is applied to the skin, the oil may form a film and water from the aqueous phase can form beads on this oily film. The beads of water may even coalesce to form larger beads of water that are visible to a user.

At least some consumers may associate the beads of water formed in this way with an inferior skin care product.

U.S. Publication Nos. 2013/0243835 and 2015/352028, filed by Tanner, et al ("Tanner") disclose O/W emulsions comprising superabsorbent polymer thickeners in the continuous aqueous phase to provide better stability over time than aqueous systems thickened with conventional thickener systems. However, the O/W emulsions in the Tanner publications have the drawbacks generally associated with O/W emulsions discussed above, including the undesirable addition of a thickener. Additionally, the Tanner applications do not recognize the benefit of putting a non-thickening superabsorbent polymer in the dispersed aqueous phase of a W/O emulsion.

U.S. Publication No. 2008/038216, filed by Zukowski, et al., discloses personal care compositions comprising W/O emulsion systems that have an internal aqueous phase present at about 1% - 75%. The '216 publication discloses that the aqueous phase visibly separates from the oil phase upon the application of a shear force (e.g., the rubbing associated with application of the composition to skin), and that the water from the aqueous phase forms droplets on the hydrophobic film formed by the oil phase. However, as pointed out above, the formation of water droplets such as those described in the '216 publication can be perceived by some consumers as an indication of an inferior skin care composition, and thus undesirable. In addition, the '216 application discloses a composition in which the droplets of the internal aqueous phase have an average size of 1 micron or less. It is believed, without being limited by theory, that droplets less than 10 microns in size may not provide the desired silky or light feel when applied to skin.

Accordingly, it would be desirable to provide a skin care composition in the form of a stable W/O HIPE emulsion that provides a desirable sensory experience during use and the perception of good moisturization, but which does not require thickeners. It would further be desirable to provide a skin care composition in the form of a stable W/O HIPE emulsion that undergoes phase separation upon application to skin, but does not form visible water droplets.

WO 2008/155228, US 2015/342864 and EP 3 114 962 disclose cosmetic compositions which are emulsions.

## SUMMARY

Disclosed is a personal care composition in the form of a water-in-oil, high internal phase emulsion, comprising more than about 74%, by volume, of an internal aqueous phase; about 0.01% to about 3%, by weight, of a superabsorbent polymer (SAP) in the aqueous phase; and less than about 26%, by volume, of a continuous oil phase.

Also disclosed is a personal care composition in the form of a water-in-oil, high internal phase emulsion, comprising more than about 74%, by volume, of an internal aqueous phase in the form of droplets having a droplet size of between about 10 and about 150 microns; about 0.1% to about 1%, by weight, of a superabsorbent polymer in the aqueous phase, the superabsorbent polymer being in the form of a multitude of particles having a number-average swollen particle size of between about 10 and about 150 microns; less than about 26%, by volume, of a continuous oil phase comprising at least one silicone elastomer; and a viscosity of between about 100 and about 10,000 Pa-s, wherein the aqueous phase does not form visible beads when applied to a target portion of skin.

Further disclosed is a method of moisturizing skin, comprising: identifying a target portion of skin where moisturization is desired; and applying a personal care composition to the target portion of skin, wherein the personal care composition is a water-in-oil high internal phase emulsion comprising more than about 74%, by volume, of an aqueous phase dispersed in an oil phase, wherein the aqueous phase is in the form of a multitude of droplets having a droplet size of between about 10 microns and about 150 microns, a superabsorbent polymer disposed in the aqueous phase, the superabsorbent polymer having a number-average dry particle size of between about 2 microns and 50 microns, and less than about 25%, by volume, of the oil phase.

## BRIEF DESCRIPTION OF THE DRAWINGS

FIG. 1 illustrates viscosity measurement for four high internal phase emulsion compositions.
FIG. 2 is a spider plot illustrating the results of a consumer test.
FIG. 3 is a spider plot illustrates the results of a consumer test.
FIG. 4A is an image of a target skin surface with a comparative composition applied thereto.
FIG. 4B is an image of a target skin surface with an example of the present composition applied thereto.

## DETAILED DESCRIPTION

In low and medium internal phase emulsions, i.e., emulsions in which the continuous phase is a larger volume fraction of the composition than the dispersed phase, the viscosity of the composition is typically controlled by adding a thickening agent(s) (e.g., a water gelling polymer) to the continuous phase. Adding thickeners to the dispersed phase generally has little or no impact on the viscosity of the emulsion. In a W/O HIPE, viscosity can be controlled, for example, by adding thickening agents such as waxes to the continuous oil phase, selecting oils that have desired rheological properties, and/or adjusting the volume fraction and/or droplet size of the dispersed phase. As with low and medium internal phase emulsions, adding a thickening agent to the dispersed phase of a W/O HIPE generally has little or no effect on the viscosity of the emulsion.

Surprisingly, it has now been discovered that incorporating relatively large superabsorbent polymer particles into the aqueous phase of a W/O HIPE can provide an improved sensory experience to a user. In particular, the present composition may feel light and/or cool when applied and be perceived as providing good moisturization. Additionally, the present composition avoids the formation of visible beads of water when applied to skin. This is desirable because the formation of water beads during application can be perceived by consumers as an indication of an inferior product.

All percentages herein are by weight of the cosmetic composition, unless specifically stated otherwise. All ratios are weight ratios, based on the weight of the relevant composition, unless specifically stated otherwise. All ranges are inclusive of narrower ranges and combinable; delineated upper and lower range limits are interchangeable to create further ranges not explicitly delineated. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at approximately 25°C and at ambient conditions, where "ambient conditions" means conditions under about 1 atmosphere of pressure and at about 50% relative humidity.

The cosmetic compositions described herein can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods. As used in the description and the appended claims, the singular forms "a," "an," and "the" are intended to include the plural forms as well, unless the context clearly indicates otherwise.

"About," as used herein, modifies a particular value, by referring to a range equal to the particular value, plus or minus twenty percent (+/- 20%).

"Apply" or "application," as used in reference to a composition, means to apply or spread the compositions of the present invention onto keratinous tissue such as the epidermis with one or more fingers and/or an implement, for example, as one would be expected to apply a cream or lotion to the facial skin.

"Aqueous phase" herein refers to the phase of an emulsion that is primarily water.

"Cosmetic" means providing a desired visual effect on an area of the human body. The visual cosmetic effect may be temporary, semi-permanent, or permanent.

"Cosmetic agent" means any substance, as well any component thereof, intended to be rubbed, poured, sprinkled, sprayed, introduced into, or otherwise applied to a mammalian body or any part thereof to provide a cosmetic effect. Cosmetic agents may include substances that are Generally Recognized as Safe ("GRAS") by the U.S. Food and Drug Administration, food additives, and materials used in non-cosmetic consumer products including over-the-counter medications. The compositions herein may optionally include one or more cosmetic agents in addition to nicotinamide riboside. Cosmetic agents may be incorporated in a cosmetic composition comprising a dermatologically acceptable carrier suitable for topical application to skin.

"Dermatologically acceptable" means that the compositions or components described are suitable for use in contact with human skin tissue without undue toxicity, incompatibility, instability, allergic response, and the like.

"Disposed" means an element is positioned in a particular place relative to another element.

"Emulsion" refers to a system comprising at least two liquid phases, wherein one of the phases is primarily water or a water miscible liquid and the other phase is primarily a liquid immiscible with water.

"Effective amount" means an amount of a compound or composition sufficient to significantly induce a desired benefit.

"External phase" and "continuous phase" refer to the phase of an emulsion in which the droplets of the internal phase are dispersed.

"Internal phase" refers to the phase of an emulsion dispersed in the continuous phase. "Discontinuous phase" and "dispersed phase" are synonymous with internal phase.

"Oil phase" means the phase of an emulsion that is primarily a liquid(s) immiscible with water.

"Personal care composition" means a composition intended to be applied to keratinous tissue to provide a health and/or appearance benefit thereto.

"Phase separation" herein refers to the separation of the aqueous and oil phases of the present composition when an applied force causes the composition to exceed its yield-point. In some instances, phase separation is accompanied by sudden decrease in viscosity. For example, phase separation may correspond to a slope of greater than -5 (e.g., between -5 and -100) on a slope plot of viscosity between the regions where the composition has a substantially constant high viscosity and a substantially constant lower viscosity.

"Skin care agent" means a cosmetic agent for regulating and/or improving a skin condition. Some nonlimiting examples of regulating and/or improving a skin condition include improving skin appearance and/or feel by providing a smoother, more even appearance and/or feel; increasing the thickness of one or more layers of the skin; improving the elasticity or resiliency of the skin; improving the firmness of the skin; reducing the oily, shiny, and/or dull appearance of skin; improving the hydration status or moisturization of the skin; improving the appearance of fine lines and/or wrinkles; improving skin exfoliation or desquamation; plumping the skin; improving skin barrier properties; improving skin tone; reducing the appearance of spots, redness or skin blotches; and/or improving the brightness, radiancy, or translucency of skin. Examples of skin care agents include moisturizing agents, conditioning agents, anti-microbials, humectants, vitamins, peptides and peptide derivatives, sugar amines, sunscreens, oil control agents, particulates, flavonoid compounds, hair growth regulators, antioxidants, phytosterols, protease inhibitors, tyrosinase inhibitors, anti-inflammatory agents, and mixtures thereof.

"Stable" means a composition is substantially unaltered in chemical state, physical homogeneity, and/or color when the composition is at a temperature of between 1° and 40° C and relative humidity of 50% for 1 week.

"Substantially," as used herein, means nearly identical and varying only in an insignificant or non-essential way. When referring to a particular property or characteristic, "substantially" means the property or characteristic does not vary by more than 10% (e.g., less than 8%, 5%, 3%, 2%, or even less than 1%).

"Superabsorbent polymer" means a polymer that is capable, in its dry state, of absorbing at least 20 times its dry weight of water. A general description of superabsorbent polymers can be found in "Absorbent Polymer Technology, Studies in Polymer Science 8" by L. Brannon-Pappas and R. Harland, published by Elsevier, 1990.

"Treatment period" means the length of time and/or frequency that a personal care composition or cosmetic agent is used. The treatment period may be a predetermined length of time and/or frequency.

"Viscosity" herein refers to dynamic viscosity unless specifically stated otherwise, and is determined according to the Viscosity Method described in more detail below.

"Yield-point" herein refers to the stress that must be applied to the present composition before it starts to flow. The yield-point of the present composition corresponds to the sudden decrease in viscosity, and can be determined according to the Viscosity Method described in more detail below.

Composition

The personal care compositions described herein are W/O HIPEs formed by dispersing a high-volume fraction aqueous phase in a low-volume fraction oil phase, for example, using methods known in the art for making HIPEs. When the aqueous phase is mixed with the oil phase, it forms a multitude of droplets dispersed throughout the oil phase. The droplets are separated from one another by a thin film of the oil phase. It can be important to control the droplet size of the aqueous phase of the present composition, since the viscosity of the composition is dependent in part on the droplet size of the aqueous phase. For example, if the droplet size decreases and the volume fraction of the aqueous phase remains constant, then the viscosity of the present composition will increase.

It will also be appreciated that the droplet size of the aqueous phase can affect the stability and/or sensory characteristics of the present composition. For example, if the droplets are too large, then the emulsion may have stability issues due to, for example, the increasing effects of buoyancy over the reduced effect of Brownian motion on the larger droplets. Also, if the droplets are too large, then they may be visible to user when the composition is applied to skin. On the other hand, if the droplets are too small, then they may not provide the desired "ball bearing effect," which is described in more detail below. In some instances, the internal phase may have an average droplet size of between 10 and 150 microns (e.g., between 20 and 100 microns, 30 and 80 microns, or even 40 - 60 microns). Droplet size can be determined using and suitable technique known in the art, such as, for example, a conventional light scattering technique.

The W/O HIPE compositions herein are formulated to provide a suitably thick cream that consumers associate with desirable moisturization properties. The thickness or viscosity may be controlled by adding a thickener to the continuous oil phase, adjusting the droplet size of the internal aqueous phase, and/or adjusting the volume fraction of the internal aqueous phase. It may be desirable to ensure that the present composition has a substantially constant viscosity, at least prior to the yield-point, across the range of conditions associated with normal use of the product to help deliver the desired sensory experience to a consumer.

The present composition includes SAP particles in the aqueous phase. In conventional compositions, SAP particles are sometimes used as thickeners. Conventional SAP thickeners typically have a dry particle size of about 1 micron or less (e.g., SEPIGEL 265, 305, and 400 from Seppic and CARBOPOL from Lubrizol). However, the SAP particles in the aqueous phase of the present composition have a relatively large dry particle size (e.g., greater than 2 microns) and do not directly affect the viscosity of the composition. In other words, the SAP particles in the present composition are not thickeners. Thus, prior to the yield-point of the present composition, there is less than a 10% (e.g., less than 8%, 5%, 3%, 2%, or even less than 1%) difference between the viscosity of the present composition and the viscosity of an identical composition that does not include SAP particles.

In some instances, the compositions herein have a viscosity of between 100 and 10,000 Pascal-seconds (Pa-s). Compositions that have a viscosity of less than 100 Pa-s may not provide the desired indication of good moisturization, and compositions that have a viscosity of more than 10,000 Pa-s may detract from the desired sensory experience (e.g., too hard to spread, feel too heavy, do not provide a silky feel). It is also desirable for the composition herein to have a substantially constant viscosity prior to the yield point, which is a general indication of good stability. If the composition exhibits significant changes in viscosity prior to the desired yield point, which may be an indication of premature phase separation or syneresis, then there is a risk that the composition may phase separate prior to use (e.g., during shipping and handling). This can be seen by a consumer as an indication of an inferior product. Accordingly, the compositions herein generally have a substantially constant viscosity prior to the yield point. In some instances, the viscosity of the present composition does not change by more than 10% standard deviation at stresses of between 0.1 Pa and 50 Pa (e.g., between 0.5 Pa and 45 Pa or between 1 Pa and 40 Pa), for a composition having a yield point of between about 40 Pa and 100 Pa (e.g., between 45 Pa and 80 Pa or between 50 Pa and 70 Pa). A suitable method of evaluating the change in viscosity or percent change in standard deviation is described in more detail in Example 2 below.

Providing a good moisturization signal to a user can be important, but it may also be important for the composition to provide a good sensory experience. For example, it may be desirable for the compositions herein to be perceived by a user as being a lighter-feeling composition during and/or after application, as opposed to the heavy and/or greasy feeling sometimes associated with low or medium internal phase ratio type W/O emulsions. The shear-thinning behavior exhibited by the present results in a lighter feel on skin. That is, the viscosity of the composition tends to decrease with increasing shear stress beyond a certain threshold, which may be predetermined. Thus, when the amount of shear force applied to the composition exceeds the yield-point of the composition, the aqueous phase will separate from the oil phase, resulting in a sudden decrease in viscosity, which may be perceived by a user as "feeling lighter". It may be desirable for the present composition to phase separate as a result of the shear force exerted by a user during normal use of the composition (e.g., when composition is applied to a target skin surface). In some instances, the phase separation can also provide a cool feeling during application due to the effect of the water present in the aqueous phase (e.g., evaporation and/or absorption). In some instances, the compositions herein may have a yield-point of between 1 and 100 Pascals (Pa) (e.g., between 20 and 90 Pa, 30 and 80 Pa, 40 and 70 Pa or even between 50 and 70 Pa).

In some instances, after the present composition phase separates, the oil phase forms a film on the skin. The oil film formed on the skin may provide a moisture barrier that inhibits transepidermal water loss, and thus improves moisturization. Additionally or alternatively, a consumer may associate the oil film with a moisturization benefit. The components present in the aqueous phase of the composition (i.e., water, SAP, and other optional ingredients) may form invisible "beads" on or in the oil film. As used herein, "invisible" means the beads are not visible to the naked eye of a person having 20/20 vision at a distance of about 30 cm under typical lighting conditions (e.g., between 500 - 10,000 lux), and "visible" means that the beads are visible under these conditions. It is believed, without being limited by theory, that the interaction of the beads formed from the aqueous phase components, especially water, with the skin of a person can provide a sensory feeling of lightness, silkiness, and/or coolness.

In some instances, the invisible beads formed when the composition phase separates can provide a "ball bearing" effect. The ball bearing effect refers to the effect produced when the beads roll along the surface of the skin without breaking, thereby reducing the friction between an applicator (e.g., finger, hand, or other device used to apply the composition as intended) and the target surface (e.g., skin), resulting in a smoother, silkier feel during application of the composition to skin. Accordingly, the beads should exhibit suitable resistance to crushing. If the aqueous beads are crushed, then they may undesirably dissociate into smaller beads during application and fail to provide the desired effect (e.g., silky, lubricious feel). In addition, it may be desirable for the aqueous beads to exhibit suitable resistance to coalescence (i.e., the formation of large beads from the merging of smaller beads), which may help avoid the undesirable formation of visible beads on the skin. However, the beads should not be so resistant to crushing/coalescence that they feel hard or rough during application. Accordingly, it can be important to incorporate a suitable amount of SAP in the aqueous phase to provide the desired amount of crush resistance. Too much SAP may make the particles feel too hard or abrasive and too little SAP may not provide sufficient particle resilience. In some instances, the present composition may include between 0.001% and 3% of SAP particles, based on the weight of the composition (e.g., 0.05% to 2%, 0.1% to 1%, 0.25% to 0.75%, or even about 0.5%).

The dispersed aqueous phase of the present composition may include water, at least one SAP, and, optionally, one or more additional water soluble, water miscible, or water dispersible components. In some instances, the aqueous phase of the present composition comprises 1% to 75% (e.g., 5% to 50% or even 10% to 25%) by weight of the composition, of components other than water. Non-limiting examples of optional additional components include water-soluble moisturizing agents, water-miscible solvents, conditioning agents, humectants, and/or other water-soluble skin care actives. In some instances, the non-water component may include glycerin.

The superabsorbent polymer present in the aqueous phase of the present composition is in the form of a multitude of water-swellable particles that have a high capacity for absorbing and retaining water and aqueous fluids. After absorbing water, the SAP particles swell but remain insoluble in the aqueous fluid, and thus are able to retain their particulate state. It is believed, without being limited by theory, that the swollen SAP particles provide a gelling effect to the droplets of the aqueous internal phase as well as the invisible aqueous beads formed during phase separation of the composition. The gelling effect provides increased structural rigidity and elasticity to the droplets and beads, thereby improving the crush resistance and coalescence resistance of the beads. This, in turn, may enable a light, cool, and silky feel during application of the present composition. The SAP particles suitable for use herein may have a dry, number-average particle size of 2 $\mu$m to 100 $\mu$m, with a median particle size of 25 $\mu$m, or even in the range of 2 $\mu$m to 40 $\mu$m with a median particle size of 12 $\mu$m. The SAP particles may have a water-absorbing capacity ranging from 20 to 2000 times their own weight (i.e., 20 g to 2000 g of water absorbed per gram of absorbent polymer), for example, 30 to 1500 times, 50 to 1000 times, or even 400 times. The water-absorbing characteristics of the SAP particles herein are defined at standard temperature and pressure conditions for distilled water. For example, the value of the water-absorbing capacity of a SAP herein can be determined by dispersing 0.5 g of polymer(s) in 150 g of distilled water, waiting 20 minutes, filtering the non-absorbed solution through a 10 $\mu$m filter for 20 minutes, and weighing the non-absorbed water to determine how much was absorbed by the polymer. In some instances, the viscosity of an SAP solution in 1% distilled water is in the range of 20 to 30 Pas (e.g., 22 to 29 Pas) at pH 4 and in the range of 23 to 28 Pas at pH 7.

Once hydrated, the SAP particles suitable for use in the present composition swell to form relatively soft beads that have a number average diameter of 10 $\mu$m to 250 $\mu$m (e.g., 15 $\mu$m to 200 $\mu$m, 20 $\mu$m - 150 $\mu$m, 25 $\mu$m - 100 $\mu$m, 40 $\mu$m - 90 $\mu$m, or even about 70 $\mu$m). A suitable method of determining the number average diameter of swollen SAP particles is described in more detail below. It can be important to tailor the swollen particle size of the SAP so that the droplet and bead sizes of the aqueous phase before and after phase separation of the composition are the desired size. Suitable methods of tailoring the swollen particle size of an SAP are known in the art.

Some non-limiting examples of SAPs that may be suitable for use herein are crosslinked sodium polyacrylates such as those sold as: Octacare™ X100, X110, and RM100 by Avecia: Flocare™ GB300 and Flosorb 500 by SNF; Liquasorb™ 1003, 1010, 1100, and 1280 by BASF; Water Lock™ G400 and G430 (INCI name: Acrylamide/Sodium Acrylate Copolymer) by Grain Processing; Aqua Keep™ 10 SH NF, Aqua Keep 10 SH NFC, sodium acrylate crosspolymer-2, by Sumitomo Seika; starches grafted by an acrylic polymer (homopolymer or copolymer) and in particular by sodium polyacrylate (INCI name: Sodium Polyacrylate Starch), such as those sold as: Sanfresh™ ST-100C, ST100MC, and IM-300MC by Sanyo Chemical Industries; hydrolysed starches grafted by an acrylic polymer (homopolymer or copolymer), in particular the acryloacrylamide/sodium acrylate copolymer, (INCI name: Starch/Acrylamide/Sodium Acrylate Copolymer) such as those sold as: Water Lock™ A-240, A-180, B-204, D-223, A-100, C-200, and D-223 by Grain Processing. A particularly suitable example of an SAP is Makimousse™ 12 and Makimousse™ 25 supplied by Kobo Products, Inc.

The present compositions include a continuous oil phase. The oil phase generally includes materials that are not miscible or soluble in water. The oils in the oil phase are typically fluid at room temperature and may be volatile or nonvolatile. "Non-volatile" means a material that exhibits a vapor pressure of less than 0.2 mm Hg at 25°C at one atmosphere and/or a material that has a boiling point at one atmosphere of at least 300°C. "Volatile" means that the material exhibits a vapor pressure of at least 0.2 mm of mercury at 20° C. Volatile oils may be used to provide a lighter feel when a heavy, greasy film is undesirable. Suitable oils include hydrocarbons, esters, amides, ethers, silicones, and mixtures thereof. In some instances, volatile and/or non-volatile silicone oils may be particularly suitable for use in the oil phase of present composition. Non-limiting examples of the foregoing oils can be found in US 2013/0243717.

The oil phase of the present composition may include one or more elastomers to help provide a pleasant feel when the composition is applied by a user. Elastomers suitable for use herein may be emulsifying or non-emulsifying silicone elastomers. For example, the present composition may include 0.1% to 15% (e.g., 0.1% to 5% or even 0.1% to 2%) of a non-emulsifying crosslinked siloxane elastomer. The indicated percentages are understood to refer to the amount of dry elastomer, as opposed to the total amount of elastomer and solvent used for example for storage and shipping. The elastomer may be a silicone elastomer powder or gel. A particularly suitable example of a non-emulsifying crosslinked siloxane elastomer is dimethicone/vinyl dimethicone crosspolymer, supplied by a variety of suppliers including Dow Corning (e.g., DC series 3091, 9040, 9041, 9045, 9506 and 9701), Momentive Performance Materials (e.g., SFE 839), Shin Etsu (e.g., KSG series 15, 16, and 18 and KSP series 100, 101, 102, 103, 104, and 105), and Grant Industries (e.g., GRANSIL line of elastomers). Other non-liming examples of elastomers, including cross-linked siloxane elastomers and processes for making them, are described in U.S. Pat. Nos. 4,970,252; 5,654,362; 5,760,116; and U.S. Publication Nos. 2008/0038216 and 2013/0243835.

The present composition may, optionally, include one or more additional components that are commonly included in cosmetic skin care compositions. The additional materials may be included in the aqueous and/or oil phase, as appropriate. Some non-limiting examples of additional components for use herein include skin care actives (e.g., sugar amines, vitamins (e.g., vitamin B3 compound, ascorbic acid, tocopherol acetate, panthenol, dexpanthenol, magnesium ascorbyl phosphate, sodium ascorbyl phosphate, retinyl propionate, and combinations thereof), sebum control agents, humectants, emollients, photosterols, hexamidine compounds, tightening agents, anti-wrinkle actives, anti-atrophy actives, flavonoids, N-acyl amino acid compounds, retinoids, peptides, particulate materials, anti-cellulite agents, desquamation actives, anti-acne actives, anti-oxidants, radical scavengers, skin conditioning agents, anti-inflammatory agents, tanning actives, skin lightening agents, botanical extracts, antimicrobial actives, antifungal actives, antibacterial actives, sensates, and combinations thereof); UV actives (e.g., 1% - 40% of an encapsulated and/or unencapsulated UV active selected from avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloy trioleate, ethyl dihydroxypropyl PABA, glyceryl aminobenzoate, lawsone with dihydroxyacetone, red petrolatum, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, isopentyl 4-methoxycinnamate, and combinations of these); emulsifiers (e.g., 0.01% to 10%); solvents (e.g., di-isopropyl adipate, butyl phthalimide, isopropyl phthalimide, phenylethyl benzoate, dicaprylyl carbonate, isopropyl lauroylsarcosinate, isononyl isononanoate, butyl octylsalicylate, dioctyl malate, dicaprylyl maleate, isopropyl isostearate, propylene glycol dicaprate, esters of C12-C15 alcohol benzoates, isosorbide diesters, and derivatives and mixtures of these); photo-stabilizers (e.g., methoxycrylene, diethylhexyl 2,6-naphthalate, diethylhexyl syringylidenemalonate, and combinations of these); fatty alcohols; vitamins; peptides; sugar amines; N-acyl amino acids; humectants; emollients; occlusive agents; particulate materials; colorants; oil control agents; and combinations of these. Additional descriptions of the foregoing examples and some additional examples can be found in US Publication Nos. 2008/0038216, 2013/0243835, 2006/0275237, 2004/0175347 and The Cosmetic, Toiletry, and Fragrance Association's The International Cosmetic Ingredient Dictionary and Handbook, Thirteenth Edition.

## Method of Use

The present composition may be used to provide a skin care benefit. For example, a skin care agent(s) may be included in the present composition to provide one or more skin health benefits such as improving the appearance of wrinkles, deep lines, fine lines, large pores, sallowness, hyperpigmentation, and/or under-eye dark circles; increasing the convolution of the dermal-epidermal border; increasing skin elasticity; improving skin hydration; and/or improving skin barrier function. The method of use may include identifying a target portion of skin (e.g., a facial skin surface such as the forehead, perioral, chin, periorbital, nose, and/or cheek) in need of treatment and/or where treatment is desired and applying a safe and effective amount of the present composition to the target portion of skin. The skin care agent(s) may be incorporated into the present composition using conventional methods for combining active agents into cosmetic compositions. Without intending to be bound by theory, it is believed that application of an effective amount of the present composition to a target portion of skin in need of treatment can provide the desired skin care benefit over the course of a treatment period.

In some instances, the target portion of skin identified for treatment may not exhibit signs of a particular skin health disorder, but a user (e.g., a relatively young user) may still wish to target such an area of skin if it is one that typically develops skin disorders later in life. In this way, the present compositions may be used as a preventative measure. In some instances, skin in the target area identified for treatment may be compared to skin not typically exposed to sunlight to determine if treatment is needed and/or desired. The composition may be applied to the target skin portion and, if desired, to the surrounding skin at least once a day, twice a day, or on a more frequent daily basis, during a treatment period. When applied twice daily, the first and second applications are separated by at least 1 to 12 hours. Typically, the composition is applied in the morning and/or in the evening before bed.

The treatment period should be of sufficient time for the skin care agent(s) in the present composition to provide the desired benefit to the target portion of skin (e.g., improve appearance, increase moisturization). The treatment period may last for at least 1 week (e.g., about 2 weeks, 4 weeks, 8 weeks, or even 12 weeks). In some instances, the treatment period will extend over multiple months (*i.e.*, 3-12 months) or multiple years. In some instances, the composition may be applied most days of the week (e.g., at least 4, 5 or 6 days a week), at least once a day or even twice a day during a treatment period of at least 2 weeks, 4 weeks, 8 weeks, or 12 weeks.

The step of applying the composition may be accomplished by localized application. In reference to application of the composition, the terms "localized", "local", or "locally" mean that the composition is delivered to the targeted area (e.g., a hyperpigmented portion of skin) while minimizing delivery to skin surfaces where treatment is not desired. The composition may be applied and lightly massaged into an area of skin. The form of the composition or the dermatologically acceptable carrier should be selected to facilitate localized application. While certain embodiments herein contemplate applying a composition locally to an area, it will be appreciated that compositions herein can be applied more generally or broadly to one or more skin surfaces.

The present composition may be applied by a variety of means, including by rubbing, wiping or dabbing with hands or fingers, or by means of an implement and/or delivery enhancement device. Non-limiting examples of implements include a sponge or sponge-tipped applicator, a swab (for example, a cotton-tipped swab), a pen optionally comprising a foam or sponge applicator, a brush, a wipe, and combinations thereof. Non-limiting examples of delivery enhancement devices include magnetic, mechanical, electrical, ultrasonic and/or other energy devices. In some instances, the composition can be spread onto the skin to facilitate the separation of the aqueous phase from the oil-phase. When the aqueous and oil phases have separated, the composition may be left on the keratinous tissue. Alternatively, the composition may be allowed to remain on the skin for 5 seconds, 10 seconds, 30 seconds, or 1 minute prior to being rubbed into the keratinous tissue.

METHODS

The specific instruments and tools described in the methods herein may be replaced by equivalent instruments and tools, which can be readily accomplished by one of ordinary skill in the art.

Viscosity Method

This method provides a suitable means of determining the viscosity and yield point of a composition. The instrument used in this method is a HAAKE RHEOSTRESS 600 brand rheometer, available from Thermo Fisher Scientific, MA. The instrument is set up to conduct an oscillatory amplitude sweep under controlled stress conditions, from 0.1 to 100 Pa and 0.1 rad/s frequency, using a cone-and-plate geometry with a 35mm diameter, 4 degree angle and a 0.140 mm gap. The temperature is set to 25 °C. The instrument protocol is set up to collect 30 data points in a logarithmic distribution where each data point is obtained from 3 measurement repetitions.

After conducting an automated plate-cone gap calibration, approximately 1.5 grams of a sample is placed on the center of the plate. The plate is positioned to provide a gap of 0.150 mm, and any excess material squeezed out of the perimeter gap of the cone is carefully trimmed away. The plate is then moved the final 0.1 mm to provide a 0.140 mm gap, and the measurement is initiated. At the end of the run, the data file is saved for subsequent plotting and analysis. The yield point is determined from the data plot and analysis.

SAP Particle Size Determination

To determine the dry particle size, the particles are first dispersed by a Light Powder Preparation for SEM Imaging. First, an ice-pick, awl, or other suitable instrument is used to poke a hole in the bottom of a foamed polystyrene cup. Using one half of a clean petri dish, a clean SEM sample stub with carbon tape is placed in the middle of the dish. A portion of the powder sample is then sprinkled into the petri dish, around the sample stub. The polystyrene cup is then placed over the sample stub and powder, and using a can of compressed air (e.g., Duster), the nozzle of the can is inserted into the hole in the bottom of the cup and puffed gently several times to disperse powder into the air. Care is taken to not shake or tip the air can upside down, as liquid residue from the propellant may contaminate the sample stub.

After sample preparation, secondary electron images are acquired at 2kV with an emission current of 7μA at room temperature on a Hitachi S-4700 FE-SEM, or equivalent, equipped with an Alto 2500 cryo-exchange chamber. Working distance was set at ~10mm for each sample. General magnifications used were 50, 100, 250, 500, 1000 and 2500X depending on the size of the particles imaged. Prior to imaging, the samples were coated with Au/Pd for 60 seconds at 10mA in the Alto 2500.

To determine the wet, swollen particle size, the particles being tested are added to DI water in a speedmixer cup at a concentration of 0.1%, by weight and mixed for 2 minutes at 3540 RPMs in a Flacktec Speedmixer. If the sample is not already neutralized, then adjust pH to 7.0 +/- 0.3, by adding NaOH. The sample should be given sufficient time and additional mixing, if necessary, to ensure the particles are fully swollen. If gel blocking occurs, which is indicated by visual agglomeration in solution, allow the sample to rest ~24 hr. Once all of the particles are fully swollen, mix again for 30 seconds at 3540 RPM.

In a separate container, prepare a suitable dye premix (e.g., 0.06 g Toluidine Dye in 1L of DI water). In a separate speedmixer cup, add the swollen particle mixture to the dye premix at 1:1 ratio. Mix for 2 minutes at 3540 rpm in the speedmixer. Apply a representative sample of the mixture to a microscope slide without a cover slide. Take appropriate measures to ensure a representative sampling of the mixture is collected, as some sampling techniques could inadvertently exclude large swollen particles from transferring to the slide. For examples, use gentle mixing (with vortex) during sampling to ensure that larger particles do not migrate to the bottom of the container.

Using transmitted light microscopy, with sufficient magnification and lighting conditions, capture images of the particles on the microscope slide. Images must be acquired within 30 min of dye addition to the particles. A Nikon AZ100 multi-zoom microscope with an AZ-Plan Fluor 5x (NA: 0.5/WD: 15mm), with attached Qimaging MicroPublisher 5.0 camera is commercially available and suitable for use herein. Magnification, lighting, and camera settings are optimized to maximize both particle contrast and spatial resolution. The optical magnification is selected based on the average size of each particle class analyzed. The resulting spatial resolutions include 1.1 to 8.9 pixels/um (5x-1 to 5x-8). Properly calibrated image analysis software (Image Pro Premier 9 suite) is used to measure the particles within the images.

EXAMPLES

Example 1 - Formulations

The formulas provided in Table 1 below represent some non-limiting examples of the W/O HIPE personal care compositions described herein.

Table 1

| Example | A | B | C | D | E | F | G | H |
|---|---|---|---|---|---|---|---|---|
| PHASE A | | | | | | | | |
| DC-9040[1] | 10.00 | - | - | 5.00 | 2.00 | | 2.00 | 2.00 |
| VELVESIL-125[2] | - | - | 7.00 | - | - | - | 2.00 | - |
| DC-3901[3] | - | 6.00 | 5.00 | - | 5.00 | 9.00 | 3.00 | 4.00 |
| Dimethicone | - | 3.50 | 6.00 | - | 5.00 | 4.00 | 8.00 | 2.00 |
| Polymethyl-silsesquioxane | - | - | - | 1.00 | - | - | - | 0.50 |
| Cyclomethicone D5 | 5.00 | - | 3.00 | 10.00 | 2.00 | 1.00 | - | 1.00 |
| KSG-210[4] | 3.00 | - | 3.00 | 3.00 | - | - | 1.00 | - |
| DC-2503 Cosmetic Wax[5] | - | - | - | - | - | 1.00 | - | - |
| KF-6038[6] | 0.25 | 0.50 | - | 0.25 | 0.50 | - | 0.40 | 0.50 |
| ABIL WE09[7] | - | - | 0.25 | - | - | 0.50 | - | - |
| $TiO_2$ particles | - | - | - | - | 0.20 | - | - | 0.10 |
| $TiO_2$ Coated Mica | - | - | - | 0.25 | - | 0.10 | 0.10 | 0.10 |
| Fragrance | 0.10 | 0.10 | 0.10 | 0.10 | 0.10 | 0.10 | 0.10 | 0.10 |
| PHASE B | | | | | | | | |
| Glycerin | 5.00 | 3.00 | 5.00 | 3.00 | 5.00 | 10.00 | 15.00 | 7.00 |
| Makimousse-25[8] | 0.50 | 0.40 | 0.75 | 0.40 | 0.50 | 0.60 | 0.80 | 0.60 |
| Dexpanthenol | 0.50 | 0.50 | 0.50 | 0.50 | 0.50 | 0.50 | 0.50 | 0.50 |
| Pentylene Glycol | - | - | 1.00 | 3.00 | 1.00 | 1.00 | - | - |
| Hexamidine Diisethionate | 0.10 | 0.10 | 0.10 | 0.10 | 0.10 | 0.10 | 0.10 | 0.10 |
| Niacinamide | 3.00 | 3.00 | 5.00 | 5.00 | 5.00 | 3.00 | 3.00 | 5.00 |
| Methylparaben | 0.20 | 0.20 | 0.20 | 0.20 | 0.20 | 0.20 | 0.20 | 0.20 |
| Ethylparaben | 0.05 | 0.05 | 0.05 | 0.05 | 0.05 | 0.05 | 0.05 | 0.05 |
| Sodium Citrate | 0.20 | 0.20 | 0.20 | 0.20 | 0.20 | 0.20 | 0.20 | 0.20 |
| Citric Acid | 0.03 | 0.03 | 0.03 | 0.03 | 0.03 | 0.03 | 0.03 | 0.03 |
| Sodium Benzoate | 0.05 | 0.05 | 0.05 | 0.05 | 0.05 | 0.05 | 0.05 | 0.05 |
| FD&C Red #40 (1%) | - | - | - | - | 0.05 | - | - | 0.05 |

(continued)

| Example | A | B | C | D | E | F | G | H |
|---|---|---|---|---|---|---|---|---|
| Water | q.s to 100 | q.s to 100 | q.s to 100 | q.s to 100 | q.s to 100 | q.s to 100 | q.s to 100 | q.s to 100 |

| |
|---|
| 1. Dimethicone crosspolymer in cyclopentasiloxane, from Dow Corning. |
| 2. Dimethicone crosspolymer in cyclopentasiloxane, from Momentive. |
| 3. Dimethicone/vinyl dimethicone crosspolymer, from Dow Corning |
| 4. Dimethicone/PEG-10/15 crosspolymer, from Shin-Etsu |
| 5. Stearyl dimethicone (and) octadecene, from Dow Corning. |
| 6. Lauryl PEG-9 polydimethylsiloxyethyl dimethicone, from Shin-Etsu |
| 7. Polyglyceryl-4 isostearate (and) cetyl PEG/PPG-10/1 dimethicone (and) hexyl laurate, from Evonik Industries. |
| 8. Sodium polyacrylate starch, from Kobo Products, Inc. |

Example compositions A to H in Table 1 are made by combining the ingredients of Phase A in a suitable container and mixing until the phase is homogenous. In a separate suitable container, the ingredients of Phase B are combined and mixed until homogeneous. Mix the SAP and glycerin, and then add the other Phase B ingredients. This should allow for a more rapid SAP swelling while reducing issues associated with gel blocking. While mixing with a suitable mixer (e.g., Anchor blade, propeller blade, or IKA T25), Phase B is slowly added to Phase A while continuing to mix Phase A. Mixing is continued until the batch is uniform. When using materials that are solids at room temperature (e.g., waxes), separately heat the Phases to a temperature that is 5 degrees above the melting point of the solid material. While maintaining this temperature, emulsify phase B into phase A as above. The resulting composition is placed into suitable containers and stored at room temperature.

Example 2

This example demonstrates that the SAP in the present composition does not substantially affect its viscosity. The four compositions tested in this example were prepared in substantially the same way as the compositions in Example 1. All four compositions are W/O HIPE, and each composition comprises about 80% by volume, based on the total volume of the composition, of an aqueous phase. Compositions A and D are comparative examples of a HIPE. Compositions B and C are representative examples of the present HIPEs.

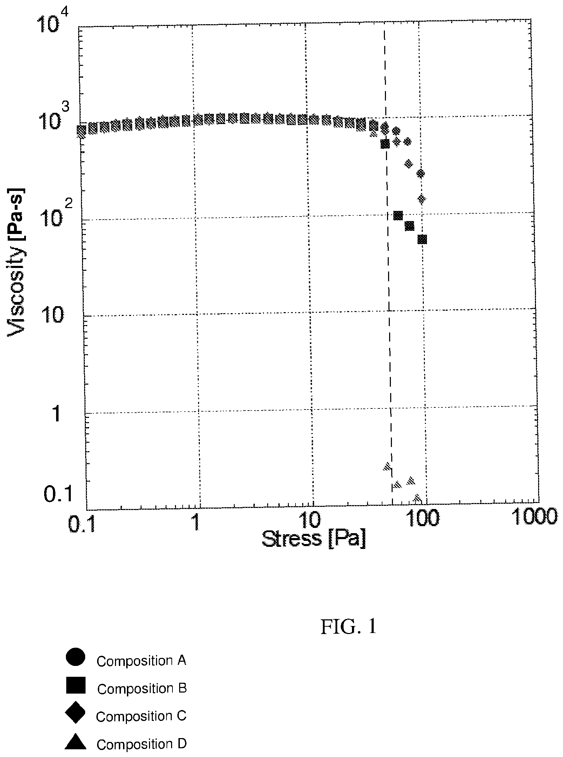

Composition A includes a relatively high amount of silicone elastomer, which is commonly used in conventional skin care compositions to provide good feel properties, but does not contain an SAP. Compositions B and C include an SAP and a silicone elastomer. Composition D includes a relatively low amount of silicone elastomer, as compared to the other compositions, and a high amount of cyclomethicone Ds, which is a non-volatile silicone commonly used in conventional skin care compositions as a carrier. Composition D also includes a silicone elastomer powder (DC 9506) instead of the silicone elastomer gel used in Examples A, B, and C (DC 9045). The results of the test are graphically illustrated in FIG. 1. Viscosity was measured according to the method described above.

Table 2

| Example | A | B | C | D |
|---|---|---|---|---|
| Water Phase: | | | | |
|     Water | qs | qs | qs | qs |
|     Makimousse-25 | ---- | 0.25 | 0.5 | ---- |
| Oil Phase: | | | | |
|     DC-9045[1] | 14.8 | 14.8 | 15.8 | ---- |
|     DC-9506[2] | | | | 2.0 |
|     Cyclomethicone D5 | | | | 16.8 |
|     KSG-210 | 1.0 | 1.0 | 1.0 | 1.0 |
|     KF-6038 | 0.2 | 0.2 | 0.2 | 0.2 |
| Total: | 100% | 100% | 100% | 100% |

(continued)

| Example | A | B | C | D |
|---|---|---|---|---|
| | | | | |
| Yield Point | ~ 50 Pa | ~ 50 Pa | ~ 50 Pa | ~ 50 Pa |
| Viscosity before yield (Pa-s) | 945.2 | 976.5 | 1014.1 | 947.2 |
| % standard deviation | 6.8 | 5.9 | 6.7 | 6.9 |
| 1. Dimethicone crosspolymer in cyclopentasiloxane, from Dow Corning. 2. Dimethicone / vinyl dimethicone crosspolymer powder, from Dow Corning. | | | | |

As can be seen in FIG. 1, compositions A, B, and C all have substantially the same viscosity prior to their respective yield points, which shows that the inclusion of the SAP does not impact the viscosity of the compositions. Further, the viscosity of each composition remained substantially constant prior to the yield point, as evidenced by their relatively low percent standard deviation from average viscosity. A constant viscosity prior to the yield point at the stresses shown in FIG. 1 indicates that the compositions should be stable prior to their intended use. The percent standard deviation in Table 2 is defined as the standard deviation of the viscosity divided by the root mean square (RMS) and multiplied by 100%.

$$\% \, Std. \, Dev. = \frac{Std. \, Dev.}{RMS} \times 100$$

The percent standard deviation calculation considers only viscosity values that are relatively constant prior to the yield point. A minimum of 3 data points per decade of stress should be used in the calculation to provide sufficient confidence. For the data shown in FIG. 1, 26 data points were used to calculate the percent standard deviation for composition A, 25 data points were used for compositions B and C, and 24 data points were used for composition D.

As illustrated in FIG. 1, composition D exhibited quicker phase separation than composition A, B, or C (i.e., composition D exhibited a sharper drop in viscosity over the same range of shear stress). It is believed, without being limited by theory, that using a silicone elastomer powder in composition D, instead of the silicone elastomer gel network used in compositions A, B, and C, provided the quicker phase separation. Compositions with a quicker phase separation may be perceived as having more of a "watery" or "light" feel during application, which is preferred by some consumers.

Example 3 - Consumer Study

This study demonstrates the superior consumer perceived benefits provided by the present composition when compared to a commercially available skin moisturizing cream. In this study, thirty women between the ages of 18 and 55 who indicated that they use facial moisturizers 3 times/week or more were recruited to assess the aesthetic attributes of two test compositions and the commercially available skin moisturizing cream. The test uses a randomized, self-dosing, split face protocol. The panelists were asked to clean their face before applying any product. The panelists were also asked to evaluate the aesthetics of the compositions during the initial product application, immediately after completion of the application, and 4 hours after application of the product.

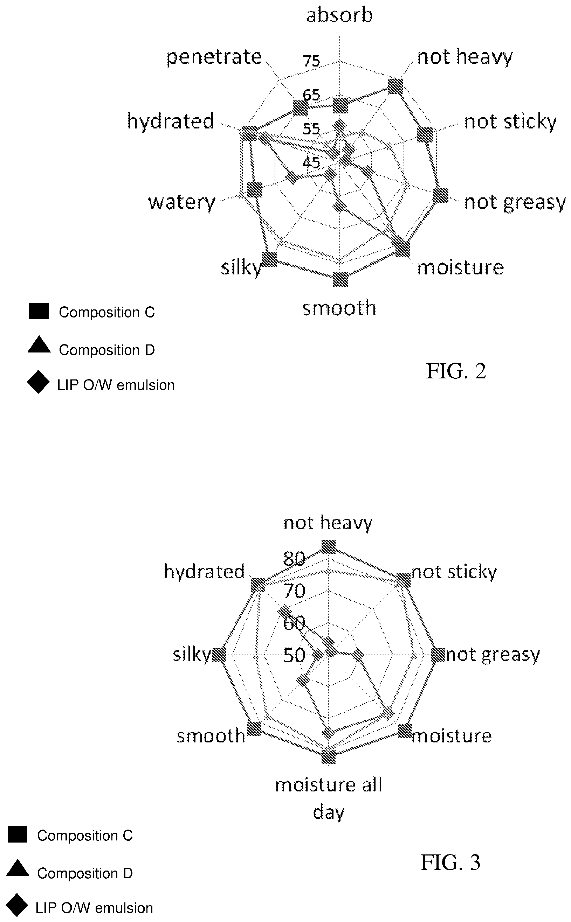

Each test composition is an SAP-containing, O/W HIPE. The formulas for the two test compositions are shown in Table 3. The commercially available skin care product is an O/W low internal phase emulsion (LIPE). The O/W LIPE is about 85%, by volume, of a continuous aqueous phase, about 70%, by weight, of water, and includes a conventional thickening agent in the aqueous phase. The results of the study are illustrated in FIGS 2 and 3, which show that the test compositions are generally perceived as having better sensory properties than a conventional skin moisturizing cream. The results also demonstrate that it may be desirable to use a silicone elastomer gel instead of or in addition to a silicone elastomer powder.

Table 3

| Example | HIPE-1 | HIPE-2 |
|---|---|---|
| Water Phase: | | |
| Water | qs | qs |

(continued)

| Example | HIPE-1 | HIPE-2 |
|---|---|---|
| Makimousse-25 | 0.48 | 0.48 |
| Glycerin | 3.95 | 3.95 |
| Methyl Paraben | 0.08 | 0.08 |
| Oil Phase: | | |
| DC-9045 | 15.30 | 8.89 |
| DC-9506 | - | 1.0 |
| KSP-102[1] | - | 0.99 |
| Cyclomethicone D5 | 4.5 | 8.90 |
| KSG-210 | 1.0 | 1.0 |
| KF-6038 | 0.17 | 0.17 |
| Total: | 100% | 100% |
| 1. Vinyl dimethicone/methicone silsesquioxane crosspolymer powder, from Shin Etsu. | | |

FIG. 2 illustrates the panelists' sensory perception of the compositions during application. As illustrated in FIG. 2, the HIPE-1 composition generally received a better sensory perception from the panelists than the HIPE-2 composition and the comparative composition, and the HIPE-2 composition generally received a better sensory perception from the panelists than the comparative composition. The comparative product was perceived by the panelists as having good moisture and hydration properties, which is expected from a conventional skin moisturizing cream. The HIPE-2 composition was perceived as watery and hydrating, possibly due to faster phase separation related to the inclusion of silicone elastomer powder in the composition.

FIG. 3 illustrates the panelists' sensory perception of the compositions 4 hours after application. As illustrated in FIG. 3, the HIPE-1 composition generally received a better sensory perception from the panelists than the HIPE-2 composition and the comparative composition, and the HIPE-2 composition generally received a better sensory perception from the panelists than the comparative composition. In particular, the HIPE-1 and HIPE-2 were perceived as not being heavy, sticky, or greasy, and yet moisturizing and hydrating.

Example 4 - Impact of SAP on Composition Aesthetics

This example demonstrates how the inclusion of a suitable SAP can improve the aesthetic properties of a personal care composition. In this example, the HIPE-1 test composition from Example 3 was compared to an identical composition that did not include the SAP. Both compositions were applied to a target skin surface in a manner typical for applying topical skin compositions. In this example, about 0.075 ml of composition was applied to the back of the left hand of a test subject. The subject spread the composition over the back of the left hand in a generally circular motion using the index finger of the right hand until a substantially uniform film was achieved. A uniform film should be achievable within 4 to 7 rotations. The hand was then positioned for imaging. A Cannon EOS 5D Camera, equipped with Sigma 50 mm, 1:2.8 DG Macro lens was use to capture the images. The lens was positioned approximately 12 cm from the hand.

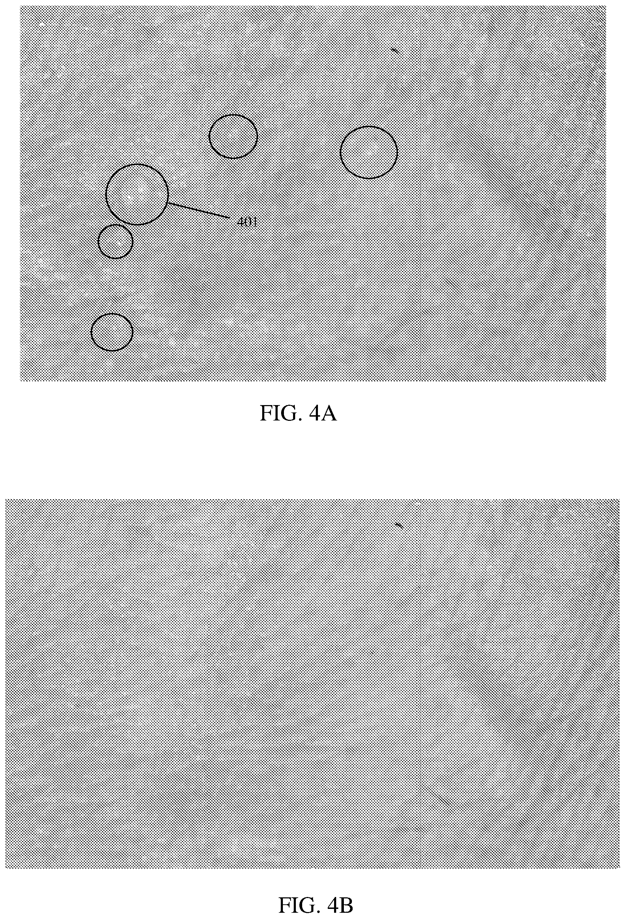

FIG. 4A shows an image of the target skin surface to which the comparative composition was applied. The circles on the image are provided to highlight the visible water beads that formed on the skin during application of the product. The largest water bead 401 measured about $2.1 \times 2.4$ mm. FIG. 4B shows an image of the target skin surface to which composition HIPE-1 was applied. As can be seen in FIG. 4B, no visible water drops formed during application of the representative composition.

Examples/Combinations

A. A personal care composition in the form of a water-in-oil, high internal phase emulsion, comprising:

a. more than about 74%, by volume, of an internal aqueous phase;

b. about 0.01% to about 3%, by weight, of a superabsorbent polymer (SAP) in the aqueous phase; and

c. less than about 26%, by volume, of a continuous oil phase.

B. The composition of paragraph A, further comprising a viscosity of between about 100 and 10,000 Pa-s.

C. The composition of paragraph B, wherein the SAP does not substantially affect the viscosity of the composition.

D. The composition of paragraph C, wherein the SAP does not change the viscosity of the composition by more than 10% as compared to an identical composition that does not include the SAP.

E. The composition of paragraph B, wherein the viscosity of the personal care composition is substantially constant prior to the yield-point.

F. The composition of any preceding paragraph, wherein the internal aqueous phase is in the form of a multitude of droplets having a droplet size of between 10 and 250 microns.

G. The composition of any preceding paragraph, wherein the SAP is in the form of a multitude of particles that have a dry particle size of between about 2 microns and 100 microns.

H. The composition of paragraph G, wherein the SAP has a swollen number-average particle size of between about 10 microns and about 250 microns.

I. The composition of any preceding paragraph, wherein the aqueous phase of the composition does not form visible beads when applied to a target portion of skin.

J. The composition of any preceding paragraph, wherein the personal care composition exhibits phase separation at a shear stress of between 10 and 100 Pa, according the Rheology Method.

K. The composition of any preceding paragraph, further comprising about 0.01 to about 10% of an emulsifier.

L. The composition of any preceding paragraph, further comprising an active or agent selected from a group consisting of sugar amines, vitamins, oil control agents, humectants, emollients, photosterols, hexamidine compounds, tightening agents, anti-wrinkle actives, anti-atrophy actives, flavonoids, N-acyl amino acid compounds, retinoids, peptides, particulate materials, anti-cellulite agents, desquamation actives, anti-acne actives, anti-oxidants, radical scavengers, conditioning agents, anti-inflammatory agents, tanning actives, skin lightening agents, botanical extracts, antimicrobial actives, antifungal actives, antibacterial actives, antiperspirant actives, sensates, preservatives, anti-dandruff actives, substantivity polymers, detersive surfactants, and combinations thereof.

M. The composition of any preceding paragraph wherein the superabsorbent polymer is selected from the group consisting of sodium polyacrylate, sodium polyacrylate starch, sodium acrylates crosspolymer-2 and mixtures thereof.

N. The composition of any preceding paragraph, further comprising a silicone elastomer.

O. The composition of paragraph N, wherein the silicone elastomer is a silicone elastomer gel.

P. A personal care composition in the form of a water-in-oil, high internal phase emulsion, comprising:

a. more than about 74%, by volume, of an internal aqueous phase in the form of droplets having a droplet size of between about 10 and about 150 microns;

b. about 0.1% to about 1%, by weight, of a superabsorbent polymer in the aqueous phase, the superabsorbent polymer being in the form of a multitude of particles having a number-average swollen particle size of between about 10 and about 150 microns;

c. less than about 26%, by volume, of a continuous oil phase comprising at least one silicone elastomer; and

d. a viscosity of between about 100 and about 10,000 Pa-s, wherein the aqueous phase does not form visible beads when applied to a target portion of skin.

Q. A method of moisturizing skin, comprising:

a. identifying a target portion of skin where moisturization is desired; and

b. applying a personal care composition to the target portion of skin, wherein the personal care composition is a water-in-oil high internal phase emulsion comprising

i. more than about 74%, by volume, of an aqueous phase dispersed in an oil phase, wherein the aqueous phase is in the form of a multitude of droplets having a droplet size of between about 10 microns and about 150 microns,
ii. a superabsorbent polymer disposed in the aqueous phase, the superabsorbent polymer having a number-average dry particle size of between about 2 microns and 50 microns, and
iii. less than about 25%, by volume, of the oil phase.

R. The method of paragraph Q, wherein the personal care composition is a skin care composition.

S. The method of paragraph Q or R, wherein the personal care composition does not form visible beads when applied to the target portion of keratinous tissue.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A personal care composition in the form of a water-in-oil, high internal phase emulsion, comprising:

a. more than about 74%, by volume, of an internal aqueous phase;
b. about 0.01% to about 3%, by weight, of a superabsorbent polymer (SAP) in the aqueous phase; and
c. less than about 26%, by volume, of a continuous oil phase.

2. The composition of Claim 1, further comprising a viscosity of between about 100 and 10,000 Pa-s, measured as disclosed herein.

3. The composition of any preceding claim, wherein the internal aqueous phase is in the form of a multitude of droplets having a droplet size of between 10 and 250 microns.

4. The composition of any preceding claim, wherein the SAP is in the form of a multitude of particles that have a dry particle size of between about 2 microns and 100 microns, measured as disclosed herein.

5. The composition of claim 4, wherein the SAP has a swollen number-average particle size of between about 10 microns and about 250 microns, measured as disclosed herein.

6. The composition of any preceding claim, further comprising about 0.01 to about 10% of an emulsifier.

7. The composition of any preceding claim wherein the superabsorbent polymer is selected from the group consisting of sodium polyacrylate, sodium polyacrylate starch, sodium acrylates crosspolymer-2 and mixtures thereof.

8. The composition of any preceding claim, further comprising a silicone elastomer.

9. A method of moisturizing skin, comprising:

a. identifying a target portion of skin where moisturization is desired; and
b. applying a personal care composition to the target portion of skin, wherein the personal care composition is a water-in-oil high internal phase emulsion comprising

i. more than about 74%, by volume, of an aqueous phase dispersed in an oil phase, wherein the aqueous phase is in the form of a multitude of droplets having a droplet size of between about 10 microns and about 150 microns,
ii. a superabsorbent polymer disposed in the aqueous phase, the superabsorbent polymer having a number-average dry particle size of between about 2 microns and 50 microns, and
iii. less than about 25%, by volume, of the oil phase.

**Patentansprüche**

1. Körperpflegezusammensetzung in der Form einer Wasser-in-Öl-Emulsion mit großem Anteil an einer inneren Phase, umfassend:

   a. zu mehr als etwa 74 Vol.- % eine innere wässrige Phase;
   b. zu etwa 0,01 Gew.-% bis etwa 3 Gew.-% ein superabsorbierendes Polymer (SAP) in der wässrigen Phase; und
   c. zu weniger als etwa 26 Vol.- % eine kontinuierliche Ölphase.

2. Zusammensetzung nach Anspruch 1, ferner eine Viskosität zwischen etwa 100 und 10.000 Pa-s, gemessen wie hierin offenbart, umfassend.

3. Zusammensetzung nach einem der vorstehenden Ansprüche, wobei die innere wässrige Phase in Form einer Vielzahl von Tröpfchen vorliegt, die eine Tröpfchengröße zwischen 10 und 250 Mikrometer aufweisen.

4. Zusammensetzung nach einem der vorstehenden Ansprüche, wobei das SAP in Form einer Vielzahl von Partikeln vorliegt, die eine Trockenpartikelgröße zwischen etwa 2 Mikrometer und 100 Mikrometer aufweisen, gemessen wie hierin offenbart.

5. Zusammensetzung nach Anspruch 4, wobei das SAP eine zahlengemittelte Partikelgröße nach Quellung zwischen etwa 10 Mikrometer und etwa 250 Mikrometer aufweist, gemessen wie hierin offenbart.

6. Zusammensetzung nach einem der vorstehenden Ansprüche, ferner zu etwa 0,01 bis etwa 10 % einen Emulgator umfassend.

7. Zusammensetzung nach einem der vorstehenden Ansprüche, wobei das Superabsorber-Polymer ausgewählt ist aus der Gruppe bestehend aus Natriumpolyacrylat, Natriumpolyacrylatstärke, Natriumacrylaten Kreuzpolymer-2 und Mischungen davon.

8. Zusammensetzung nach einem der vorstehenden Ansprüche, ferner ein Silikonelastomer umfassend.

9. Verfahren zum Befeuchten von Haut, umfassend:

   a. Identifizieren eines Zielbereichs der Haut, wo eine Befeuchtung gewünscht ist; und
   b. Aufbringen einer Körperpflegezusammensetzung auf den Zielbereich der Haut, wobei die Körperpflegezusammensetzung eine Wasser-in-Öl-Emulsion mit großem Anteil an einer inneren Phase ist, umfassend:

   i. zu mehr als etwa 74 Vol.-% eine wässrige Phase, die in einer Ölphase dispergiert ist, wobei die wässrige Phase in Form einer Vielzahl von Tröpfchen vorliegt, die eine Tröpfchengröße zwischen etwa 10 Mikrometer und etwa 150 Mikrometer aufweisen,
   ii. ein superabsorbierendes Polymer, das in der wässrigen Phase angeordnet ist, wobei das superabsorbierende Polymer ein Zahlenmittel der Trockenpartikelgröße zwischen etwa 2 Mikrometer und 50 Mikrometer aufweist, und
   iii. zu weniger als etwa 25 Vol.-% die Ölphase.

**Revendications**

1. Composition de soins personnels sous la forme d'une émulsion à phase interne élevée, eau-dans-huile, comprenant :

a. plus d'environ 74 %, en volume, d'une phase aqueuse interne ;

b. environ 0,01 % à environ 3 %, en poids, d'un polymère superabsorbant (PSA) dans la phase aqueuse ; et

c. moins d'environ 26 %, en volume, d'une phase huileuse continue.

**2.** Composition selon la revendication 1, comprenant en outre une viscosité comprise entre environ 100 et 10 000 Pa·s, mesurée comme décrit ici.

**3.** Composition selon une quelconque revendication précédente, dans laquelle la phase aqueuse interne est sous la forme d'une multitude de gouttelettes ayant une taille de gouttelette comprise entre 10 et 250 micromètres.

**4.** Composition selon une quelconque revendication précédente, dans laquelle le PSA est sous la forme d'une multitude de particules qui ont une taille de particule sèche comprise entre environ 2 micromètres et 100 micromètres, mesurée comme décrit ici.

**5.** Composition selon la revendication 4, dans laquelle le PSA a une taille de particule gonflée moyenne en nombre entre environ 10 micromètres et environ 250 micromètres, mesurée comme décrit ici.

**6.** Composition selon une quelconque revendication précédente, comprenant en outre environ 0,01 à environ 10 % d'un émulsifiant.

**7.** Composition selon l'une quelconque des revendications précédentes, dans laquelle le polymère superabsorbant est choisi dans le groupe constitué de polyacrylate de sodium, amidon de polyacrylate de sodium, polymère réticulé-2 d'acrylates de sodium et mélanges de ceux-ci.

**8.** Composition selon une quelconque revendication précédente, comprenant en outre un élastomère de silicone.

**9.** Procédé d'hydratation de la peau, comprenant :

a. l'identification d'une partie cible de peau où une hydratation est souhaitée ; et

b. l'application d'une composition de soins personnels à la partie cible de peau, dans lequel la composition de soins personnels est une émulsion à phase interne élevée eau-dans-huile comprenant

i. plus d'environ 74 %, en volume, d'une phase aqueuse dispersée dans une phase huileuse, dans lequel la phase aqueuse est sous la forme d'une multitude de gouttelettes ayant une taille de gouttelette comprise entre environ 10 micromètres et environ 150 micromètres,

ii. un polymère superabsorbant disposé dans la phase aqueuse, le polymère superabsorbant ayant une taille de particule sèche moyenne en nombre comprise entre environ 2 micromètres et 50 micromètres, et

iii. moins d'environ 25 %, en volume, de la phase huileuse.

FIG. 1

● Composition A
■ Composition B
◆ Composition C
▲ Composition D

FIG. 2

FIG. 3

FIG. 4A

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130243835 A
- US 2015352028 A, Tanner
- US 2008038216 A, Zukowski
- WO 2008155228 A
- US 2015342864 A
- EP 3114962 A
- US 20130243717 A
- US 4970252 A
- US 5654362 A
- US 5760116 A
- US 20080038216 A
- US 20060275237 A
- US 20040175347 A

**Non-patent literature cited in the description**

- **L. BRANNON-PAPPAS ; R. HARLAND.** Absorbent Polymer Technology, Studies in Polymer Science 8. Elsevier, 1990
- The International Cosmetic Ingredient Dictionary and Handbook. The Cosmetic, Toiletry, and Fragrance Association